(19) Europäisches Patentamt
European Patent Office
Office européen des brevets

(11) Veröffentlichungsnummer: **0 293 748 B1**

(12) **EUROPÄISCHE PATENTSCHRIFT**

(45) Veröffentlichungstag der Patentschrift: **29.01.92**

(51) Int. Cl.⁵: **C07D 307/58**

(21) Anmeldenummer: **88108365.3**

(22) Anmeldetag: **26.05.88**

(54) **Verfahren zur Herstellung von 2(5H)-Furanonen.**

(30) Priorität: **03.06.87 DE 3718527**

(43) Veröffentlichungstag der Anmeldung:
**07.12.88 Patentblatt 88/49**

(45) Bekanntmachung des Hinweises auf die Patenterteilung:
**29.01.92 Patentblatt 92/05**

(84) Benannte Vertragsstaaten:
**CH DE FR LI NL**

(56) Entgegenhaltungen:
**US-A- 2 809 203**

**BULLETIN DE LA SOCIETE CHIMIOUE DE FRANCE, 1960, Seiten 1241-1242, Paris, FR; S. DUCHER: "Hydrolyse des oxo-4 butanoates d'alcoyle"**

**SYNTHESIS, Nr. 8, August 1985, Seiten 786-788, Stuttgart, DE; J.-A.H. NÄSMAN et al.: "An improved one-pot preparation of 2-furanones"**

(73) Patentinhaber: **BASF Aktiengesellschaft**
**Carl-Bosch-Strasse 38**
**W-6700 Ludwigshafen(DE)**

(72) Erfinder: **Fischer, Rolf, Dr.**
**Bergstrasse 98**
**W-6900 Heidelberg(DE)**
Erfinder: **Frank, Juergen, Dr.**
**Hirschbrunnenweg 82**
**W-6830 Schwetzingen(DE)**
Erfinder: **Merger, Franz, Dr.**
**Max-Slevogt-Strasse 25**
**W-6710 Frankenthal(DE)**
Erfinder: **Roeper, Michael, Dr.**
**Albert-Schweitzer-Strasse 10**
**W-6706 Wachenheim(DE)**
Erfinder: **Vagt, Uwe, Dr.**
**Paul-Neumann-Strasse 44**
**W-6720 Speyer(DE)**

**EP 0 293 748 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von 2(5H)-Furanonen durch Cyclisierung von 3-Formylcarbonsäureestern oder -carbonsäuren.

Es ist bekannt, daß sich 3-Methyl-2(5H)-furanon (3-Methyl-2,5-dihydrofuran-2-on) z.B. ausgehend von 1-Hydroxy-3-oxobutan (Ch. J. Cavallito, Th. H. Haskell, Journ. Amer. Chem. Soc. 68, 2332 (1946)), 2-Methyl-3-butencarbonsäure (M. Franck-Neumann, Ch. Berger, Bull. Soc. Chim. France 1968, 4067), 3-Methylbutyrolacton (S. Inayama, T. Kawamata, Chem. Pharm. Bull. 1973, 461) oder 2-Butin-1-ol (A. Cowell, J.K. Stille, Tetrahedron Letters 1979, 133) herstellen läßt.

4-Methyl-2(5H)-furanon ist z.B. durch Hydrierung von Methylmaleinsäureanhydrid mit Metallhydriden zugänglich (Can.J.Chem. 58, 1980, 2484).

Die angegebenen Synthesewege sind nicht befriedigend, da sie entweder zahlreiche Stufen beinhalten oder von schwer zugänglichen Ausgangsstoffen ausgehen. Der Erfindung lag daher die Aufgabe zugrunde, einen einfachen Zugang zu gegebenenfalls substituierten 2(5H)-Furanonen zu ermöglichen.

Demgemäß wurde ein Verfahren zur Herstellung von 2(5H)-Furanonen der allgemeinen Formel I

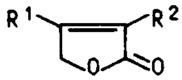 (I),

in der die Reste $R^1$ und $R^2$ Wasserstoff, $C_1$- bis $C_6$-Alkyl-, $C_5$- oder $C_6$-Cycloalkyl-, $C_7$- bis $C_{12}$-Aralkyl- oder Arylreste bedeuten, gefunden, das dadurch gekennzeichnet ist, daß man 3-Formylcarbonsäureester oder -carbonsäuren der allgemeinen Formel II

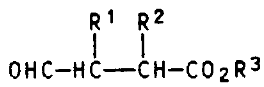 (II),

in der $R^3$ Wasserstoff oder einen $C_1$- bis $C_{12}$-Alkyl-, $C_5$- oder $C_6$-Cycloalkyl-, $C_7$- bis $C_{12}$-Aralkyl- oder einen Arylrest bedeutet, in Gegenwart von sauren Katalysatoren auf Temperaturen von 200 bis 450 °C erhitzt.

Dieser Befund überrascht, da bekannt war dass 3-Formylpropionsäure und deren Alkylester in saurem Milieu bei 100 °C 5-Hydroxy- bzw. 5-Alkoxy-2-oxotetrahydrofuran bilden, wobei im ersten Fall Dimerisation zum Bis(2-oxotetrahydrofur-5-yl)ether eintritt (Bull. Soc. Chim. Fr. 1960, 1241/2).

Die erfindungsgemäße Umsetzung läßt sich für den Fall der Herstellung von 4-Ethyl-2(5H)-furanon aus 3-Formylvaleriansäuremethylester durch folgende Reaktionsgleichung wiedergeben:

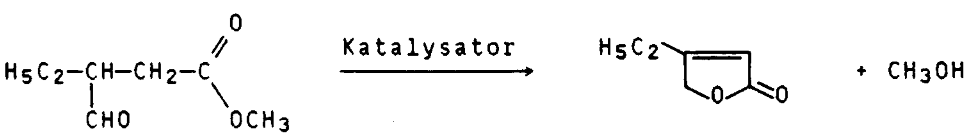


Als saure Katalysatoren können homogen gelöste Katalysatoren oder insbesondere heterogene, z.B. im Reaktionsgemisch suspendierte Katalysatoren, verwendet werden. Beispielsweise kommen Mineralsäuren wie Schwefelsäure, Phosphorsäure, Salzsäure, Salpetersäure, Bromwasserstoff oder Sulfonsäuren wie Benzolsulfonsäure oder p-Toluolsulfonsäure in Betracht. Es können auch saure Ionenaustauscher in ihrer H-Form, die z.B. aus Sulfonsäuregruppen enthaltendem vernetztem Polystyrol aufgebaut sind, verwendet werden.

Bevorzugte Katalysatoren sind sauer wirkende Oxide von Elementen der III. und IV. Hauptgruppe sowie der Nebengruppen IV bis VI des Periodischen Systems. Beispielhaft seien Siliciumdioxid, z.B. in Form von Kieselgel, Kieselgur oder Quarz, Bortrioxid, Aluminiumtrioxid, Titandioxid, Zirkondioxid, Vanadiumpentoxid, Chrom-, Molybdän- oder Wolframoxide sowie Gemische dieser Oxide genannt.

Weitere bevorzugte Katalysatoren sind oberflächenreiche silikatische Materialien wie natürliche oder synthetische Zeolithe. Solche Zeolithe sind z.B. in C.K. Hersh, Molecular Sieves, Reinhold Publishing Company, New York (1961), Seite 21, Tabelle 3-1 oder in Ullmanns Enzyklopädie der technischen Chemie, 4. Auflage, Band 17, Seiten 9 bis 16 und Band 24, Seiten 575 bis 577 aufgeführt. Besonders geeignet sind

Zeolithe aus der Chabasit-, Mordenit- und vor allem Faujasit- und Pentasilgruppe. Vorteilhafte synthetische Zeolithe sind solche mit einem ausgesprochen kieselsäurereichen Zeolith-Gitter wie Zeolithe X, Y und Pentasilzeolithe, z.B. Alumino-, Boro- oder Eisensilikatzeolithe vom Pentasiltyp, die mit Übergangsmetallen, z.B. mit Edelmetallen wie Pd, seltenen Erdmetallen wie Ce, La oder mit Eisen, Zink oder Wolfram, dotiert sein können. Solche Pentasil-Zeolithe und ihre Herstellung sind z.B. in der DE-OS 35 06 632 beschrieben.

Als Ausgangsstoffe II kommen 3-Formylcarbonsäuren und deren Ester in Frage. Da bei der Herstellung dieser Ausgangsverbindungen häufig die Ester anfallen, z.B. bei der Hydroformylierung von Pentensäureestern oder Acrylestern (vgl. EP-A-31 100 oder J. Falbe, N. Huppes, Brennstoffchemie, Bd. 48, Seiten 46-52, 1967) können vorteilhaft die Ester direkt für die erfindungsgemäße Umsetzung verwendet werden.

Als Reste $R^1$ und $R^2$ in den Ausgangsstoffen II kommen Wasserstoff, $C_1$- bis $C_8$-, insbesondere verzweigte oder unverzweigte $C_1$- bis $C_4$-Alkylreste, $C_5$- oder $C_6$-Cycloalkylreste, $C_7$- bis $C_{12}$-Aralkylreste oder Arylreste in Betracht. Alkyl- und Cycloalkylreste sind z.B. Methyl-, Ethyl-, Propyl-, Isopropyl-, Butyl-, sek.-Butyl-, tert.-Butyl-, Cyclopentyl- oder Cyclohexylreste. Aralkyl- oder Arylreste sind z.B. Benzyl-, Phenylethyl-, Phenyl-, Tolyl-, Xylyl- oder Naphthylreste, wobei die aromatischen Kerne jeweils unter den Reaktionsbedingungen inerte Gruppen wie $C_1$-$C_4$-Alkyl- oder $C_1$-$C_4$-Alkoxyreste, Halogen wie Fluor, Chlor oder Brom oder $C_1$-$C_4$-Halogenalkylreste tragen können. Besonders bevorzugt stellen $R^1$ und $R^2$ Wasserstoff oder Alkylreste dar.

Der Rest $R^3$ ist sehr breit variierbar und steht für Wasserstoff, $C_1$- bis $C_{12}$-Alkyl, $C_5$- oder $C_6$-Cycloalkyl, $C_7$- bis $C_{12}$-Aralkyl wie Phenylethyl oder Benzyl sowie Aryl, z.B. Phenyl oder substituiertes Phenyl. Zweckmäßigerweise wählt man den Rest $R^3$ so, daß der entstehende Alkohol leicht aus dem Reaktionsgemisch, z.B. destillativ, abgetrennt werden kann. Vorteilhaft sind niedermolekulare Alkylreste mit 1 bis 6 C-Atomen, Cyclohexyl, Cyclopentyl, Phenyl oder Benzyl.

Als Ausgangsverbindungen II kommen z.B. 3-Formylbuttersäure, 3-Formylvaleriansäure, 3-Formylpropionsäure, 2-Methyl-3-formyl-propionsäure, insbesondere in Form ihrer Methyl-, Ethyl-, Propyl-, Butyl-, Cyclohexyl-, Phenyl- und Benzylester in Frage.

Die Umsetzung der 3-Formylcarbonsäureester kann kontinuierlich oder diskontinuierlich in der Flüssigphase oder in der Gasphase bei Atmosphärendruck oder darüber, vorteilhaft bei Drücken von 1 bis 50, insbesondere 1 bis 5 bar durchgeführt werden. Die Reaktionstemperatur beträgt 200 bis 450 °C, insbesondere um 350 °C.

Die Umsetzung in der Flüssigphase wird beispielsweise so durchgeführt, daß man in Gegenwart eines suspendierten Festbettkatalysators oder eines homogen gelösten Katalysators auf die gewünschte Reaktionstemperatur erhitzt. Die Menge des Katalysators kann bei homogen gelösten Mineral-oder Sulfonsäuren bei 0,002 bis 0,25 mol je Mol Ausgangsstoff II liegen. Bei heterogenen, suspendierten Katalysatoren liegt sie im allgemeinen bei 0,5 bis 20 Gew.% Katalysator, bezogen auf den Ausgangsstoff II.

Die Aufarbeitung kann in an sich bekannter Weise erfolgen, indem man den Katalysator z.B. durch Filtration oder Neutralisation entfernt und das Reaktionsgemisch zur Gewinnung der Lactone I fraktionierend destilliert.

Bei Umsetzungen in der Gasphase, die häufig bevorzugt sind, geht man zweckmäßigerweise so vor, daß man den Ausgangsstoff II zunächst verdampft und dann, gegebenenfalls zusammen mit einem Inertgas wie Stickstoff, Kohlendioxid oder Argon, bei der gewünschten Reaktionstemperatur gasförmig über einen fest angeordneten oder in auf- und abwirbelnder Bewegung befindlichen Katalysator leitet. Die Katalysatorbelastung kann vorteilhaft bei 0,1 bis 10, insbesondere 0,1 bis 5 g II pro Gramm Katalysator und Stunde liegen. Der Reaktionsaustrag kann mittels geeigneter Kühlvorrichtung kondensiert und anschließend durch fraktionierende Destillation in an sich bekannter Weise aufgearbeitet werden. Unumgesetzter Formylester II kann in die Synthesestufe zurückgeführt werden.

Im Hinblick auf Selektivität und Katalysatorstandzeit der erfindungsgemäßen Umsetzung kann es günstig sein, die Umsetzung in Gegenwart von Lösungsmitteln vorzunehmen. Hierbei kommen Wasser, Alkohole wie z.B. Methanol, Ethanol, Propanol, Isopropanol, Butanole, Kohlenwasserstoffe, wie Pentan, Hexan, Petrolether, halogenierte Kohlenwasserstoffe wie z.B. Methylenchlorid, Chloroform, 1,2-Dichlorethan, Ether wie z.B. Methyl-tert.-butylether, Dioxan, Tetrahydrofuran oder Gemische dieser Lösungsmittel in Frage. Bevorzugte Lösungsmittel sind Wasser und/oder $C_1$- bis $C_4$-Alkohole.

Das Molverhältnis von Formylcarbonsäureester II zu Lösungsmittel kann beispielsweise 1:0,1 bis 1:50, insbesondere 1:0,5 bis 1:20, betragen.

Die nach dem erfindungsgemäßen Verfahren herstellbaren 2(5H)-Furanone I stellen wertvolle Zwischenprodukte z.B. zur Herstellung von Herbiziden (FR-PS 2 475 547) und $C_5$-Phosphoryliden für Wittig-Reaktionen (Aust. J. Chem. 1974, 1491-1503) dar.

Das Verfahren nach der Erfindung soll an folgenden Beispielen veranschaulicht werden.

Beispiel 1

Pro Stunde wurden 10 g 3-Formylvaleriansäuremethylester bei Normaldruck in einen Verdampfer gepumpt und von dort gasförmig zusammen mit 3 l Stickstoff bei 350 °C über 5 g eines $SiO_2$-Katalysators geleitet. Der Katalysator befand sich in einer Rohrschlange (VAA, 1 m Länge, 6 mm Durchmesser), die in einem Heißluftofen konstant bei 350 °C gehalten wurde. Die gasförmigen Reaktorausträge wurden kondensiert, gewogen und gaschromatographisch analysiert.

Innerhalb von 8 Stunden Reaktionszeit wurden 77 g Reaktionsprodukt erhalten. Die gaschromatographische Analyse (Flächen-%) ergab, daß 80 % -Ethyl-2(5H)-furanon, 9 % 3-Formylvaleriansäuremethylester und 11 % unbekannte Produkte enthalten waren. Durch fraktionierende Destillation wurden hieraus 46,7 g 4-Ethyl-2(5H)-furanon vom Siedepunkt 77 bis 78 °C/0,35 mbar (75 % der Theorie) und 6,5 g unumgesetzter 3-Formylvaleriansäuremethylester vom Siedepunkt 38 bis 40 °C/0,3 mbar (8 % der Theorie) gewonnen.

Beispiele 2 bis 7

Entsprechend Beispiel 1 wurden die in folgender Tabelle aufgeführten Umsetzungen durchgeführt.

Tabelle

Umsetzung von 3-Formylcarbonsäuremethylestern

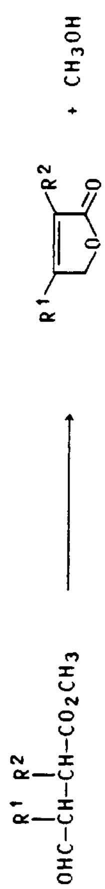


II

I

| Bsp. | $R^1$ | $R^2$ | Katalysator | Katalysatormenge (g) | Temperatur (°C) | Reaktionszeit (h) | Reaktionsaustrag[a)] Endstoff I (%) | Edukt (%) | Sonstige (%) |
|---|---|---|---|---|---|---|---|---|---|
| 2 | H | $CH_3$ | $SiO_2$ | 5 | 400 | 3,5 | 49 | 51 | - |
| 3 | $C_2H_5$ | H | $TiO_2$ | 5 | 350 | 3 | 67 | 26 | 7 |
| 4 | $C_2H_5$ | H | $\gamma$-$Al_2O_3$ | 5 | 300 | 1 | 69 | 19 | 12 |
| 5[b)] | $CH_3$ | H | Y-Zeolith[c)] | 5 | 350 | 2 | 72 | 21 | 7 |
| 6[b)] | H | H | " | 5 | 400 | 2 | 15 | 76 | 9 |
| 7 | H | $CH_3$ | Y-Zeolith | 5 | 400 | 2 | 64 | 26 | 10 |

a) gaschromatographische Analyse, Angabe in Flächen-%

b) 11 g Ausgangsstoff pro Stunde

c) 0,1 bis 1 mm Splitt

Vergleichsbeispiel

Die Umsetzung wurde wie im Beispiel 7 beschrieben, aber mit Lävulinsäuremethylester(4-Ketopentansäuremethylester) anstelle von 4-Formylpropionsäuremethylester durchgeführt. Die gaschromatographische

Analyse ergab nach 2 Stunden Reaktionszeit, daß neben 46 % unumgesetztem Lävulinester, 14 % 5-Methyl-2(3H)-furanon entstanden waren. 5-Methyl-2(5H)-furanon wurde nicht beobachtet.

Bei dem in den Beispielen verwendeten Zeolith handelte es sich um einen Y-Zeolith der in bekannter Weise durch Ionenaustausch der handelsüblichen Na-Form mittels wäßriger Ammoniumnitratlösung in die H-Form überführt worden war.

Beispiel 8

Pro Stunde wurden rund 10 g 3-Formylvaleriansäure in einen Verdampfer gepumpt und von dort gasförmig zusammen mit 3 l Stickstoff bei 350 °C über 5 g eines $SiO_2$-Katalysators geleitet.

Nach 4 Stunden Reaktionszeit wurde 2 Stunden bilanziert. In diesem Zeitraum wurden 20,7 g 3-Formylvaleriansäure zugefahren und 20,1 g Reaktionsaustrag aufgefangen. Durch fraktionierende Destillation wurden hieraus 15,5 g 4-Ethyl-2(5H)-furanon gewonnen (86 % d.Th.).

## Patentansprüche

1. Verfahren zur Herstellung von 2(5H)-Furanonen der allgemeinen Formel I

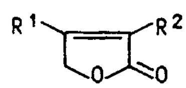

(I),

in der die Reste $R^1$ und $R^2$ Wasserstoff, $C_1$- bis $C_6$-Alkyl-, $C_5$- oder $C_6$-Cycloalkyl-, $C_7$- bis $C_{12}$-Aralkyl- oder Arylreste bedeuten, dadurch gekennzeichnet, daß man 3-Formylcarbonsäureester oder -carbonsäuren der allgemeinen Formel II

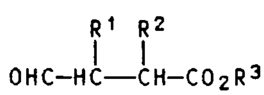

(II),

in der $R^3$ Wasserstoff oder einen $C_1$- bis $C_{12}$-Alkyl-, $C_5$- oder $C_6$-Cycloalkyl-, $C_7$- bis $C_{12}$-Aralkyl- oder einen Arylrest bedeutet, in Gegenwart von sauren Katalysatoren auf Temperaturen von 200 bis 450 °C erhitzt.

2. Verfahren nach Anspruch 1, dadurch gekennzeichnet, daß man als saure Katalysatoren Oxide von Bor, Aluminium, Silicium, Phosphor oder von Metallen der Nebengruppen IV bis VI des Periodischen Systems verwendet.

3. Verfahren nach Anspruch 1, dadurch gekennzeichnet, daß man als saure Katalysatoren Siliciumdioxid, Aluminiumtrioxid, Bortrioxid, Titandioxid, Vanadiumpentoxid, Chromtrioxid oder Gemische dieser Verbindungen verwendet.

4. Verfahren nach Anspruch 1, dadurch gekennzeichnet, daß man als saure Katalysatoren natürliche oder synthetische Zeolithe verwendet.

5. Verfahren nach Anspruch 1, dadurch gekennzeichnet, daß man X-, Y- oder Pentasilzeolithe verwendet.

6. Verfahren nach Anspruch 1, dadurch gekennzeichnet, daß die Reste $R^1$ und $R^2$ einen $C_1$- bis $C_6$-Alkylrest und $R^3$ einen $C_1$- bis $C_{12}$-Alkylrest bedeuten.

7. Verfahren nach Anspruch 1, dadurch gekennzeichnet, daß man die Lactonisierung in Gegenwart von Wasser und/oder $C_1$- bis $C_4$-Alkoholen durchführt.

## Claims

1. A process for the preparation of a 2(5H)-furanone of the formula I

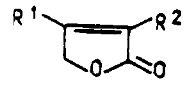

where $R^1$ and $R^2$ are each hydrogen, $C_1$-$C_6$-alkyl, $C_5$- or $C_6$-cycloalkyl, $C_7$-$C_{12}$-aralkyl or aryl, wherein a 3-formylcarboxylate or 3-formylcarboxylic acid of the formula II

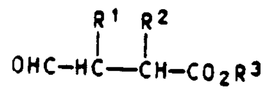

where $R^3$ is hydrogen, $C_1$-$C_{12}$-alkyl, $C_5$- or $C_6$-cycloalkyl, $C_7$-$C_{12}$-aralkyl or aryl, is heated to 200-450 ° C in the presence of an acidic catalyst.

**2.** A process as claimed in claim 1, wherein the acidic catalyst used is an oxide of boron, of aluminum, of silicon, of phosphorus or of a metal of subgroups IV to VI of the Periodic Table.

**3.** A process as claimed in claim 1, wherein the acidic catalyst used is silica, aluminum trioxide, boron trioxide, titanium trioxide, vanadium pentoxide, chromium trioxide or a mixture of these compounds.

**4.** A process as claimed in claim 1, wherein the acidic catalyst used is natural or synthetic zeolite.

**5.** A process as claimed in claim 1, wherein an X, Y or pentasil zeolite is used.

**6.** A process as claimed in claim 1, wherein $R^1$ and $R^2$ are each $C_1$-$C_6$-alkyl and $R^3$ is $C_1$-$C_{12}$-alkyl.

**7.** A process as claimed in claim 1, wherein the lactonization is carried out in the presence of water and/or a $C_1$-$C_4$-alcohol.

**Revendications**

**1.** Procédé de préparation de 2(5H)-furanones de formule générale I

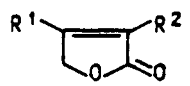 (I),

dans laquelle les restes $R^1$ et $R^2$ représentent des restes hydrogène, alkyle en $C_1$-$C_6$, cycloalkyle en $C_6$ ou $C_6$, aralkyle en $C_7$ à $C_{12}$ ou aryle, caractérisé en ce qu'on chauffe à des températures de 200 à 450 ° C, en présence de catalyseurs acides, des esters ou des acides 3-formylcarboxyliques de formule générale II

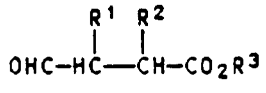 (II),

dans laquelle $R^3$ représente l'hydrogène ou un reste alkyle en $C_1$-$C_{12}$, cycloalkyle en $C_5$ ou $C_6$, aralkyle en $C_7$-$C_{12}$ ou aryle.

**2.** Procédé selon la revendication 1, caractérisé en ce qu'on utilise comme catalyseurs acides des oxydes du bore, de l'aluminium, du silicium, du phosphore ou des métaux des groupes IVb à VIb de la classification périodique.

**3.** Procédé selon la revendication 1, caractérisé en ce qu'on utilise comme catalyseurs acides du dioxyde de silicium, du trioxyde d'aluminium, du trioxyde de bore, du dioxyde de titane, du pentoxyde de

vanadium, du trioxyde de chrome ou des mélanges de ces composés.

**4.** Procédé selon la revendication 1, caractérisé en ce qu'on utilise comme catalyseurs acides des zéolithes naturelles ou synthétiques.

**5.** Procédé selon la revendication 1, caractérisé en ce qu'on utilise des zéolithes de type X, Y ou pentasil.

**6.** Procédé selon la revendication 1, caractérisé en ce que les restes $R^1$ et $R^2$ représentent un reste alkyle en $C_1$-$C_6$ et $R^3$ un reste alkyle en $C_1$-$C_{12}$.

**7.** Procédé selon la revendication 1, caractérisé en ce qu on effectue la lactonisation en présence d'eau et/ou d'alcools en $C_1$-$C_4$.